(19) Europäisches Patentamt
European Patent Office
Office européen des brevets

(11) **EP 2 077 989 B1**

(12) **EUROPÄISCHE PATENTSCHRIFT**

(45) Veröffentlichungstag und Bekanntmachung des Hinweises auf die Patenterteilung:
**01.02.2012 Patentblatt 2012/05**

(21) Anmeldenummer: **07820444.3**

(22) Anmeldetag: **21.09.2007**

(51) Int Cl.:
***C07C 213/02*** (2006.01) ***C07C 217/08*** (2006.01)

(86) Internationale Anmeldenummer:
**PCT/EP2007/060017**

(87) Internationale Veröffentlichungsnummer:
**WO 2008/037659 (03.04.2008 Gazette 2008/14)**

(54) **VERFAHREN ZUR HERSTELLUNG VON 2,2'-AMINOETHOXYETHANOL IN ELEKTRO-QUALITÄT**

METHOD FOR PRODUCING ELECTRONIC GRADE 2,2'-AMINOETHOXYETHANOL

PROCÉDÉ POUR LA PRODUCTION DE 2,2'-AMINOÉTHOXYÉTHANOL DE QUALITÉ ÉLECTRONIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **28.09.2006 EP 06121405**

(43) Veröffentlichungstag der Anmeldung:
**15.07.2009 Patentblatt 2009/29**

(73) Patentinhaber: **BASF SE**
**67056 Ludwigshafen (DE)**

(72) Erfinder:
• **SCHMIDTKE, Helmut**
**64625 Bensheim (DE)**
• **RUDLOFF, Martin**
**67161 Gönnheim (DE)**

(56) Entgegenhaltungen:
**EP-A- 0 510 382     WO-A-03/076386**
**WO-A-2007/036496**

**Beschreibung**

Die Erfindung betrifft ein Verfahren zur Herstellung von 2,2'-Aminoethoxyethanol (im Folgenden abgekürzt mit ADG bezeichnet) in Elektronik-Qualität. ADG wird durch Umsetzung von Diethylenglykol mit Ammoniak in Gegenwart eines Katalysators hergestellt (EP 510 382). Dabei entsteht Morpholin als Koppelprodukt (WO 0376386). Das Reaktionsgemisch wird in der Regel destillativ in mehreren Stufen aufgetrennt, wobei nach Abtrennung von Ammoniak, Wasser und Nebenkomponenten ein ADG sowie ein Morpholin enthaltender Strom gewonnen werden.

Die Reindestillation des ADG-haltigen Stromes erfolgt in einer Seitenstromkolonne, in der leichtsiedende Nebenkomponenten über Kopf abgetrennt und schwersiedende Nebenkomponenten im Kolonnensumpf ausgetragen werden. Als Seitenstrom wird Rein-ADG mit einem ADG-Gehalt von mindestens 98,0 Gew.-% erhalten.

Die Apparate in der Anlage zur Gewinnung von Rein-ADG (insbesondere Reaktoren und Trennkolonnen) bestehen in der Regel aus austenitischen Edelstählen, insbesondere mit den Werkstoffnummern 1.4541 und Nr. 1.4571.

Für Elektronik-Anwendungen, beispielsweise zum Reinigen von Leiterplatten, werden jedoch an Rein-ADG spezielle Spezifikationsanforderungen bezüglich der zulässigen Restgehalte an Metallen gestellt. ADG in Elektronik-Qualität muss typischerweise die folgenden Spezifikationsanforderungen erfüllen:

ADG-Gehalt: min. 98,00 Gew.-%, Diethylenglykol-Gehalt: max. 0,40 Gew.-%, WasserGehalt: max. 0,20 Gew.-% (Bestimmungsmethode Karl Fischer nach DIN 51777), Farbzahl max. 20 APHA (nach DIN EN 1557), ein Maximalgehalt an Aluminium, Arsen, Gold, Bor, Calcium, Cadmium, Cobalt, Chrom, Kupfer, Kalium, Magnesium, Mangan, Nickel, Antimon, Zinn und Titan von jeweils 20 ppb sowie ein Maximal-Gehalt an Eisen, Natrium und Zink von jeweils 30 ppb, jeweils bestimmt nach ICP-MS (ion coupled plasma-mass spectrometry).

Es wurde jedoch gefunden, dass bei der Produktion von ADG unter gewöhnlichen Prozessbedingungen die obigen Spezifikationsanforderungen nicht zuverlässig erreicht werden können. Besondere Einschränkungen bestehen bei der Verarbeitung von Diethylenglykol, welches - beispielsweise durch Lagerung und Transport - mit mehr als 50 ppb durch die oben genannten Metallionen vorverunreinigt ist.

Es war daher Aufgabe der Erfindung, ein Verfahren zur Verfügung zu stellen, wonach ADG in Elektronik-Qualität in bestehenden Anlagen gewonnen werden kann, ohne umfangreiche, kostenaufwändige apparative und/oder verfahrenstechnische Zusatzmaßnahmen. Insbesondere sollten keine Änderungen bei der Umsetzung des Eduktes am Katalysator und den zwischengeschalteten Destillationsstufen vorgenommen werden müssen.

Die Lösung besteht in einem Verfahren zur Herstellung von ADG in Elektronik-Qualität durch Umsetzung von Diethylenglykol mit Ammoniak in einem Reaktor in Gegenwart eines Katalysators unter Erhalt eines Reaktionsgemisches, woraus ein Roh-2,2'-ADG-Strom abtrennt wird, der in einer Reinkolonne weiter destillativ gereinigt wird, das dadurch gekennzeichnet ist, dass aus der Reinkolonne ein Seitenstrom abgezogen wird, enthaltend ADG in Elektronik-Qualität, indem das Diethylenglykol vor der Zuführung desselben zum Reaktor über einen Filter geleitet wird, der für Feststoffpartikel mit einer maximalen Partikelgröße von ≤ 1,5 $\mu$m einen Abscheidegrad von mindestens 99 % gewährleistet.

Der Zulauf des Diethylenglykols zur Produktionsanlage kann insbesondere durch vorgelagerte Produktionsschritte, Lagerung und Transport mit Metallionen vorverunreinigt sein, typischerweise mit Verunreinigungen in einer Konzentration von bis zu 2000 $\mu$g/l, oder auch 400 $\mu$g/l oder auch 0 bis 100 $\mu$g/l.

Die Erfinder haben erkannt, dass eine wesentliche Verbesserung bezüglich des Restgehaltes an spezifikationsschädlichen Komponenten, insbesondere Kationen, im ADG möglich ist, wenn in der Zuleitung des Diethylenglykols zum Reaktor ein Hochleistungsfilter eingebaut wird, der zur Abscheidung von Feststoffpartikeln mit einer maximalen Partikelgröße ≤1,5 $\mu$m, bevorzugt mit einer maximalen Partikelgröße ≤ 1 $\mu$m, geeignet ist.

Besonders bevorzugt wird ein Filter, dessen Abscheideverhalten sich durch folgende Kennwerte charakterisieren lässt:

Für Feststoffpartikel mit einer maximalen Partikelgröße von 0,2 $\mu$m wird ein Abscheidegrad von mindestens 60 % erreicht, für Feststoffpartikel mit einer maximalen Partikelgröße von 0,6 $\mu$m ein Abscheidegrad von mindestens 90 %, für Feststoffpartikel mit einer maximalen Partikelgröße von 0,8 $\mu$m ein Abscheidegrad von mindestens 95 %, für Feststoffpartikel mit einer maximalen Partikelgröße von 1,5 $\mu$m ein Abscheidegrad von mindestens 99 % und für Feststoffpartikel mit einer maximalen Partikelgröße von 5,0 $\mu$m ein Abscheidegrad von mindestens 99,98 %.

Um das Abscheideverhalten zu charakterisieren, lässt sich die maximale Partikelgröße zum Beispiel mittels statischer Lichtstreuung bestimmen.

Bevorzugt wird als Filter ein Beutelfilter aus Polypropylen, insbesondere mit einem Volumen von 30 bis 40 l, insbesondere von 32 l, der in einem Abscheidebehälter eingesetzt und von oben nach unten durchströmt wird. Die Zurückhaltung der Verunreinigungen erfolgt an der Innenseite des Filters.

In einer bevorzugten Verfahrensführung werden neben der Filtration des der Synthese zugeführten Eduktes Diethylenglykol auch besondere Betriebsbedingungen in der zur ADG-Reindestillation eingesetzten Kolonne gewählt, die am Ende der Prozesskette angeordnet ist. In dieser Kolonne wird das aufdestillierte ADG in Elektronik-Qualität in flüssigem Zustand als Seitenstrom abgetrennt, während die organischen Nebenkomponenten mit einem Siedepunkt > 235°C (bei 1,013 bar) im Sumpf und die organischen Verbindungen mit einem Siedepunkt < 222°C (bei 1,013 bar) über Kopf der Kolonne ausgeschleust werden. Die Kolonne wird bevorzugt bei einem Druck von 0,005 bis 0,2 bar betrieben.

Die Zulaufstelle des ADG-haltigen Stromes befindet sich - bezogen auf die theoretischen Trennstufen - oberhalb der Kolonnenmitte. Der gegenüberliegende flüssige Seitenabzug des Reinproduktes befindet sich 1 bis 30, in besonderen Fällen 10 bis 25 theoretische Trennstufen oberhalb der Zulaufstelle im Verstärkungsteil der Kolonne.

Bei dem erfindungsgemäßen Verfahren werden die Trennstufen, die sich zwischen dem Zulauf des Roh-ADG-Stromes und dem Abzug des Seitenstromes, enthaltend ADG in Elektronik-Qualität, befinden, gegenüber bekannten Verfahren, bevorzugt mit erhöhter Flüssigkeitsbelastung $w_L$ betrieben (bei ansonsten gleich bleibender Geschwindigkeit des aufsteigenden Dampfes). Die Flüssigkeitsbelastung $w_L$ stellt das Verhältnis zwischen dem Volumenstrom der auf die Trennstufen herabrieselnden Flüssigkeit $V_L$ und der freien Querschnittsfläche A der Trennstufen dar. Die Flüssigkeitsbelastung $w_L$ besitzt die Dimension einer Geschwindigkeit

$$w_L\,[m/s] = \dot{V}_L\,[m^3/s]\,/\,A[m^2]$$

Die Geschwindigkeit des aufsteigenden Dampfes $w_D$ wird aus dem Mengenstrom des am Kopf der Kolonne kondensierten Brüden $m_L$, der Dichte des Dampfes $\rho_D$ und der freien Querschnittsfläche A der Trennstufen gebildet:

$$w_D[m/s] = \dot{m}_L\,[kg/s]\,/\,\rho_D\,[kg/m^3]\,/\,A[m^2]$$

Bei dem erfindungsgemäßen Verfahren konnten die im Edukt der Produktionsanlagen eingetragenen und am Zulauf der Reinkolonne noch in höheren Anteilen vorhandenen metallischen Verunreinigungen bei einem erhöhten Verhältnis von Flüssigkeitsbelastung $w_L$ zu Dampfgeschwindigkeit $w_D$ und zwar bevorzugt von $w_L / w_D > 0,160$, in besonderen Fällen von $w_L / w_D > 0,170$, vom Produkt ferngehalten werden.

Besonders bevorzugt wird das Verfahren mit Rückführung betrieben:

Dabei wird das im Prozess nicht zu Wertprodukt umgesetzte Diethylenglykol im Sumpf der ADG-Reinkolonne abgezogen und dem frischen Eduktstrom, enthaltend Roh-ADG, vor dem Filter zugemischt. Diese Verfahrensschaltung ermöglicht eine erhöhte Ausschleusung von metallischen Verunreinigungen, insbesondere nach Abstellungen der Anlage, bei denen durch das Öffnen von Anlagenkomponenten zusätzliche Verunreinigungen in das ansonsten geschlossene System eingetragen werden.

Ausführungsbeispiele

Das erfindungsgemäße Verfahren wurde unter den genannten Bedingungen in kontinuierlicher Fahrweise in einer Produktionsanlage eingesetzt.

Es wurde eine ADG-Reinkolonne mit 44 theoretischen Trennstufen eingesetzt, wobei zwischen der Zuführung des Eduktstromes, enthaltend Roh-ADG, und dem Abzug des Seitenstromes, enthaltend Rein-ADG in Elektronik-Qualität, 9 theoretische Trennstufen angeordnet waren. Es wurde das Verhältnis von Flüssigkeitsbelastung und Dampfgeschwindigkeit für die theoretischen Trennstufen zwischen der Zuführung des Eduktstroms und dem Abzug des Seitenstromes variiert und dessen Einfluss sowie der Einfluss des Einsatzes eines Filters im Eduktweg auf die Eisen-Restkonzentration im Produkt untersucht.

Beispiele 1 bis 3

Es wurde ein Eduktstrom enthaltend Diethylenglykol mit 300 ppb Eisen eingesetzt.

Beispiel 1

Das Verhältnis von Flüssigkeitsbelastung und Dampfgeschwindigkeit wurde auf einen Wert von 0,165 eingestellt. Das Verfahren wurde ohne mechanischen Filter im Eduktweg betrieben.

Der Produktstrom, enthaltend Rein-ADG, hatte eine Eisenkonzentration von 50 ppb.

Beispiel 2

Das Verfahren wurde analog Beispiel 1, ebenfalls ohne mechanischen Filter im Eduktweg, jedoch mit einem Verhältnis von Flüssigkeitsbelastung zu Dampfgeschwindigkeit von 0,185, betrieben.

Die Eisen-Restkonzentration im ADG-Produktstrom betrug 27 ppb.

Beispiel 3

Das Verfahren wurde analog Beispiel 1 und 2 betrieben, jedoch mit einem Verhältnis von Flüssigkeitsbelastung zu Dampfgeschwindigkeit von 0,183, zusätzlich mit einem mechanischen Hochleistungsfilter im Eduktweg, und zwar einem Beutelfilter aus Polypropylen mit einem Volumen von 32 l mit der folgenden Abscheidecharakteristik:

für Feststoffpartikel mit einer maximalen Partikelgröße von 0,2 $\mu$m ein Abscheidegrad von mindestens 60 %, für Feststoffpartikel mit einer maximalen Partikelgröße von 0,6 $\mu$m ein Abscheidegrad von 90 %, für Feststoffpartikel mit einer maximalen Partikelgröße von 0,8 $\mu$m ein Abscheidegrad von mindestens 95 %, für Feststoffpartikel mit einer maximalen Partikelgröße von 1,5 $\mu$m ein Abscheidegrad von mindestens 99 % und für Feststoffpartikel mit einer maximalen Partikelgröße von 5,0 $\mu$m ein Abscheidegrad von mindestens 99,98 %.

Die Konzentration an Eisen im Rein-ADG betrug in diesem Fall 9 ppb.

Beispiele 4 bis 6

Die Beispiele 4 bis 6 wurden mit einer Eisenkonzentration im Eduktstrom von 345 ppb durchgeführt. In sämtlichen Beispielen 4 bis 6 wurde jeweils ein mechanischer Hochleistungsfilter im Eduktweg, entsprechend der Beschreibung zum vorstehenden Beispiel 3, eingesetzt.

Die Beispiele 4 bis 6 unterscheiden sich durch unterschiedliche Werte für das Verhältnis aus Flüssigkeitsbelastung und Dampfgeschwindigkeit bei ansonsten unveränderten Betriebsbedingungen

Beispiel 4

Das Verhältnis von Flüssigkeitsbelastung zu Dampfgeschwindigkeit betrug 0,178. Dabei wurde eine Restkonzentration an Eisen im Rein-ADG von 7 ppb erzielt.

Beispiel 5

Das Verhältnis von Flüssigkeitsbelastung und Dampfgeschwindigkeit betrug 0,155, entsprechend der Betriebsweise nach dem Stand der Technik. Durch Einsatz des Hochleistungsfilters im Eduktweg konnte dabei die Restverunreinigung an Eisen im Rein-ADG auf 28 ppb begrenzt werden.

Beispiel 6

Das Verhältnis von Flüssigkeitsbelastung zu Dampfgeschwindigkeit wurde zu 0,175 eingestellt. Die Restverunreinigung im Rein-ADG-Strom an Eisen betrug 8 ppb.

**Patentansprüche**

1. Verfahren zur Herstellung von 2,2'-Aminoethoxyethanol in Elektronik-Qualität durch Umsetzung von Diethylenglykol mit Ammoniak in einem Reaktor in Gegenwart eines Katalysators unter Erhalt eines Reaktionsgemisches, woraus ein Roh-2,2'-Aminoethoxyethanol-Strom abtrennt wird, der in einer Reinkolonne weiter destillativ gereinigt wird, **dadurch gekennzeichnet, dass** aus der Reinkolonne ein Seitenstrom abgezogen wird, enthaltend 2,2'-Aminoethoxyethanol in Elektronik-Qualität, indem das Diethylenglykol vor der Zuführung desselben zum Reaktor über einen Filter geleitet wird, der für Feststoffpartikel mit einer maximalen Partikelgröße von $\leq$ 1,5 $\mu$m einen Abscheidegrad von mindestens 99 % gewährleistet.

2. Verfahren nach Anspruch 1, **dadurch gekennzeichnet, dass** das Diethylenglykol über einen Filter geleitet wird, der für Feststoffpartikel mit einer maximalen Partikelgröße von $\leq$ 1,0 $\mu$m einen Abscheidegrad von mindestens 99 % gewährleistet.

**3.** Verfahren nach Anspruch 1 oder 2, **dadurch gekennzeichnet, dass** der Filter ein Beutelfilter aus Polypropylen ist.

**4.** Verfahren nach einem der Ansprüche 1 bis 3, **dadurch gekennzeichnet, dass** der Abzug des Seitenstromes aus der Reinkolonne, enthaltend 2,2'-Aminoethoxyethanol in Elektronik-Qualität, 1 bis 30, insbesondere 15 bis 25 theoretische Trennstufen oberhalb der Zulaufstelle für den Roh-2,2'-Amino-ethoxyethanolstrom angeordnet ist, und dass die Trennstufen, die sich zwischen dem Zulauf des Roh-2,2'-Aminoethoxyethanolstromes und dem Abzug des Seitenstromes, enthaltend 2,2'-Aminoethoxyethanol in Elektronik-Qualität, befinden, mit einem Verhältnis von Flüssigkeitsbelastung zu Dampfgeschwindigkeit > 0,160, betrieben werden.

**5.** Verfahren nach Anspruch 4, **dadurch gekennzeichnet, dass** der Abzug des Seitenstromes aus der Reinkolonne, enthaltend 2,2'-Aminoethoxyethanol in Elektronik-Qualität, 1 bis 30, insbesondere 15 bis 25 theoretische Trennstufen oberhalb der Zulaufstelle für den Roh-2,2'-Aminoethoxyethanolstrom angeordnet ist, und dass die Trennstufen, die sich zwischen dem Zulauf des Roh-2,2'-Amino-ethoxyethanolstromes und dem Abzug des Seitenstromes, enthaltend 2,2'-Aminoethoxyethanol in Elektronik-Qualität, befinden, mit einem Verhältnis von Flüssigkeitsbelastung zu Dampfgeschwindigkeit > 0,170 betrieben werden.

**6.** Verfahren nach einem der Ansprüche 1 bis 5, **dadurch gekennzeichnet, dass** der Sumpfstrom aus ADG-Reinkolonne abgezogen und dem frischen Eduktstrom, enthaltend Roh-2,2'-Aminoethoxyethanol, vor dem Filter zugemischt wird.

## Claims

**1.** A process for preparing electronics-grade 2,2'-aminoethoxyethanol by reacting diethylene glycol with ammonia in the presence of a catalyst in a reactor to give a reaction mixture from which a crude 2,2'-aminoethoxyethanol stream is separated off and is purified further by distillation in a pure column, wherein a sidestream comprising electronics-grade 2,2'-aminoethoxyethanol is taken off from the pure column as a result of the diethylene glycol being passed through a filter which ensures a degree of removal of at least 99% for solid particles having a maximum particle size of $\leq 1.5\ \mu$m before the diethylene glycol is fed into the reactor.

**2.** The process according to claim 1, wherein the diethylene glycol is passed through a filter which ensures a degree of removal of at least 99% for solid particles having a maximum particle size of $\leq 1.0\ \mu$m.

**3.** The process according to claim 1 or 2, wherein the filter is a bag filter made of polypropylene.

**4.** The process according to any of claims 1 to 3, wherein the offtake for the sidestream from the pure column comprising electronics-grade 2,2'-amino-ethoxyethanol is located from 1 to 30, in particular from 15 to 25, theoretical plates above the feed point for the crude 2,2'-aminoethoxyethanol stream and the separation stages located between the inlet for the crude 2,2'-aminoethoxyethanol stream and the offtake for the sidestream comprising electronics-grade 2,2'-aminoethoxyethanol are operated at a ratio of liquid loading to vapor velocity of > 0.160.

**5.** The process according to claim 4, wherein the offtake for the sidestream from the pure column comprising electronics-grade 2,2'-aminoethoxyethanol is located from 1 to 30, in particular from 15 to 25, theoretical plates above the feed point for the crude 2,2'-aminoethoxyethanol stream and the separation stages located between the inlet for the crude 2,2'-aminoethoxyethanol stream and the offtake for the sidestream comprising electronics-grade 2,2'-aminoethoxyethanol are operated at a ratio of liquid loading to vapor velocity of > 0.170.

**6.** The process according to any of claims 1 to 5, wherein the bottom stream from the pure ADG column is taken off and mixed into the fresh feedstream comprising crude 2,2'-aminoethoxyethanol upstream of the filter.

## Revendications

**1.** Procédé de préparation de 2,2'-aminoéthoxyéthanol de qualité électronique par transformation de diéthylèneglycol avec de l'ammoniac dans un réacteur en présence d'un catalyseur avec obtention d'un mélange réactionnel, dont on sépare un flux de 2,2'-aminoéthoxyéthanol brut, qui est purifié davantage par distillation dans une colonne de produit pur, **caractérisé en ce qu'**on soutire de la colonne de produit pur un flux latéral, contenant du 2,2'-aminoéthoxyéthanol de qualité électronique, **en ce que** le diéthylèneglycol est guidé avant son alimentation dans le réacteur sur un filtre qui assure un degré de séparation d'au moins 99% pour des particules solides présentant une grosseur maximale de particule ≤ 1,5 μm.

2. Procédé selon la revendication 1, **caractérisé en ce que** le diéthylèneglycol est guidé sur un filtre qui assure un degré de séparation d'au moins 99% pour des particules solides présentant une grosseur maximale de particule ≤ 1,0 μm.

3. Procédé selon la revendication 1 ou 2, **caractérisé en ce que** le filtre est un filtre à sac en polypropylène.

4. Procédé selon l'une quelconque des revendications 1 à 3, **caractérisé en ce que** le soutirage du flux latéral de la colonne de produit pur, contenant du 2,2'-aminoéthoxyéthanol de qualité électronique, est situé 1 à 30, en particulier 15 à 25 étages de séparation théoriques au-dessus du site d'alimentation du flux de 2,2'-aminoéthoxyéthanol brut et **en ce que** les étages de séparation qui se trouvent entre l'alimentation du flux de 2,2'-aminoéthoxyéthanol brut et le soutirage du flux latéral, contenant du 2,2'-aminoéthoxyéthanol de qualité électronique, sont exploités avec un rapport de charge en liquide à vitesse de la vapeur > 0,160.

5. Procédé selon la revendication 4, **caractérisé en ce que** le soutirage du flux latéral de la colonne de produit pur, contenant du 2,2'-aminoéthoxyéthanol de qualité électronique, est situé 1 à 30, en particulier 15 à 25 étages de séparation théoriques au-dessus du site d'alimentation du flux de 2,2'-aminoéthoxyéthanol brut et **en ce que** les étages de séparation qui se trouvent entre l'alimentation du flux de 2,2'-aminoéthoxyéthanol brut et le soutirage du flux latéral, contenant du 2,2'-aminoéthoxyéthanol de qualité électronique, sont exploités avec un rapport de charge en liquide à vitesse de la vapeur > 0,170.

6. Procédé selon l'une quelconque des revendications 1 à 5, **caractérisé en ce que** le flux de fond de la colonne d'ADG pur est soutiré et mélangé au flux de produit de départ frais, contenant du 2,2'-aminoéthoxyéthanol brut, en amont du filtre.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 510382 A
- WO 0376386 A